# EUROPEAN PATENT SPECIFICATION

(11) **EP 2 547 312 B1**
(45) Date of publication and mention of the grant of the patent: **06.12.2017**
(21) Application number: 11722159.8
(22) Date of filing: 15.03.2011
(51) Int. Cl.: A61K 8/02, A61K 8/63, A61K 8/68, A61Q 19/08, A61K 8/36, A61K 8/34, A61K 8/49

(54) **MICROPARTICLES OF EPIDERMAL LIPIDS CONTAINING POLYALCOHOL FOR THE PREPARATION OF COSMETIC AND PHARMACEUTICAL COMPOSITIONS**
MIKROPARTIKEL AUS EPIDERMALEN LIPIDEN MIT POLYALKOHOL ZUR HERSTELLUNG KOSMETISCHER ODER PHARMAZEUTISCHER ZUSAMMENSETZUNGEN
MICROPARTICULES DE LIPIDES ÉPIDERMIQUES CONTENANT UN POLYALCOOL POUR LA PRÉPARATION DE COMPOSITIONS COSMÉTIQUES OU PHARMACEUTIQUES

(30) Priority: 16.03.2010 IT BL20100007
(43) Date of publication of application: 23.01.2013
(73) Proprietor: Unifarco S.p.A., 32035 Santa Giustina (BL) (IT)
(72) Inventor: BARATTO, Giovanni, I-32035 Santa Giustina (BL) (IT); SEMENZATO, Alessandra, I-35131 Padova (PD) (IT); CALICETI, Paolo, I-35131 Padova (PD) (IT)
(74) Representative: Asensio, Raffaella Consuelo
(86) International application number: PCT/IB2011/000542
(87) International publication number: WO 2011/114214

(56) References cited:
- EP-A1- 1 875 896
- EP-A1- 1 969 953
- US-A1- 2006 251 708
- AMATO L ET AL: "Corneometers researches confirm the efficacy of a new product containing ceramide 3, cholesterol and fatty acids in the treatment of secondary cutaneous xerosis. Results of a pilot study", MINERVA DERMATOLOGICA, EDIZIONI MINERVA MEDICA, TURIN, IT, vol. 137, no. 4, 1 January 2002 (2002-01-01), pages 281-286, XP009145005, ISSN: 0026-4741
- TÜRKAN ELDEM ET AL: "Optimization of Spray-Dried and -Congealed Lipid Micropellets and Characterization of Their Surface Morphology by Scanning Electron Microscopy", PHARMACEUTICAL RESEARCH, KLUWER ACADEMIC PUBLISHERS-PLENUM PUBLISHERS, NL, vol. 8, no. 1, 1 January 1991 (1991-01-01) , pages 47-54, XP019371140, ISSN: 1573-904X, DOI: DOI:10.1023/A:1015874121860
- FREITAS C ET AL: "Spray-drying of solid lipid nanoparticles (SLN^T^M)", EUROPEAN JOURNAL OF PHARMACEUTICS AND BIOPHARMACEUTICS, ELSEVIER SCIENCE PUBLISHERS B.V., AMSTERDAM, NL, vol. 46, no. 2, 1 September 1998 (1998-09-01), pages 145-151, XP004257035, ISSN: 0939-6411, DOI: 10.1016/S0939-6411(97)00172-0

## Description

### Field of invention

The present invention relates to lipid-based microparticles for preparing cosmetic or pharmaceutical compositions for topical use.

### Background of the invention

In the cosmetic and pharmaceutical fields, the application of epidermal lipids, such as cholesterol, ceramides and fatty acids, is useful to reconstruct damaged skin barriers, to prevent dehydration and to attenuate and prevent roughness.

Usually, in cosmetic formulations having an emollient effect, lipids in non-micronized form only create a relatively impermeable surface layer with occlusive properties varying according to the polarity characteristics of the lipids; this layer can be easily removed by common cleansers and mechanical agents, and thus lose its protective effectiveness.

The occlusive power of solid particles of lipid nature has been described in the literature concerning nanoparticles prepared by *High Pressure Homogenization (HPH); in vitro*, these particles have shown occlusive properties depending on their size, crystalline state and lipid concentration [S.A. Wissing, R.H. Muller, Int. J. Pharm. 242 (2002) 377-379; S.A. Wissing, A. Lippacher, R.H. Muller, J. Cosmet. Sci. 52 (2001) 313-324].

A known technique for the preparation of solid microparticles is *spray-drying,* in which a solution or suspension containing one or more active ingredients and carriers in a suitable aqueous and/or organic solvent, is mixed with an inert gas, usually nitrogen, and run through a suitably heated nozzle (spray nozzle), to form a cloud of droplets from which the solvent evaporates, leaving solid particles having a size of less than 1000 µm, usually from 1 to 100 µm.

Solid nanoparticles containing lipids and prepared by a spray-drying method using carbohydrates such as mannitol or threalose in the spraying medium are described by C. Freitas and R.H. Müller in European Journal of Pharmaceutics and Biopharmaceutics 46 (1998) 145-151. Nevertheless, microparticles compo.sed of ceramides, cholesterol and fatty acids are hardly prepared by *spray-drying,* because the physical-chemical properties of aqueous-organic solutions of these lipids will cause microparticles to form agglomerates and have no homogeneous particle-size distribution. Therefore, advantages might be obtained from the provision of a method of preparing solid microparticles based on epidermal lipids that might obviate this drawback.

### Description of the figures

Figure 1: size distribution, by volume, of two different batches of solid lipid microparticles of the invention.
Figure 2: change of viscosity with increasing shear strain in an emulsion containing microparticles of the invention, an emulsion containing epidermal lipids in non-microparticle form and in a lipid-free emulsion.
Figure 3: Wilcoxon test results on an emulsion containing microparticles of the invention and on an emulsion containing no epidermal lipids.
Figure 4: Mann-Witney test and U-test results on an emulsion containing microparticles of the invention and on an emulsion containing no epidermal lipids.

### Disclosure of the invention

It has now been found that microparticles based on epidermal lipids may be conveniently prepared by *spray-drying* by adding a polyalcohol, particularly mannitol, to the starting solution or suspension.

In a first aspect, the present invention relates to solid microparticles consisting of epidermal lipids and a polyalcohol selected from mannitol, threalose and xylitol, wherein the amount of the polyalcohol ranges from 5% to 20% by weight based on the weight of the lipids and wherein the epidermal lipids are a mixture of cholesterol, one or more ceramides and one or more fatty acids at a weight ratio of 1:1:1. Preferably, epidermal lipids are a mixture composed of cholesterol, ceramide 3 and one or more C₁₆-C₁₈ fatty acids.

The term polyalcohol designates a polyalcohol selected from mannitol, trehalose and xylitol; in a preferred embodiment of the invention, the polyalcohol is mannitol, and its amount is up to 15% by weight based on the weight of epidermal lipids.

More preferably, the solid microparticles of the invention are composed of cholesterol, ceramide 3, a mixture of C₁₆-C₁₈ fatty acids and mannitol at a weight ratio of 1:1:1:0.45.

In a second aspect, the invention relates to a process for preparing solid microparticles which includes spray-drying of an alcoholic solution containing 1 to 20% (w/v) epidermal lipids and 0,1 to 5% (w/v) of mannitol, wherein the epidermal lipids are a mixture of cholesterol, ceramides and one or more fatty acids at a weight ratio of 1:1:1. The alcoholic solution may be an ethanol/water mixture; in the ethanol/water mixture, the volume ratio of ethanol and water may range from 70/30 to 99/1. In a preferred embodiment, the starting solution contains 9% (w/v) epidermal lipids and 1.35% (w/v) mannitol in an 84:16 ethanol/water mixture (v/v). In a particularly preferred embodiment, the epidermal lipids are cholesterol, ceramide 3 and a mixture of C₁₆-C₁₈ fatty acids at a weight ratio of 1:1:1.

The *spray-drying* process of the invention may be carried out using equipments known to the skilled person, taking care to heat the lipids/polyalcohol/water/alcohol mixture beforehand, to a temperature ranging from 70 to 90°C, preferably from 70 to 85°C, more preferably from 80 to 85°C, to obtain a homogeneous solution. The temperature of the spray nozzle (inlet temperature) will be appropriately regulated according to the type of equipment being used. The spray gas, the flow rate and the pressure may be also selected by the skilled person. Usually, nitrogen is used, with a flow rate from 20 to 120 Kg/h, preferably 80 Kg/h, at a pressure from 0.1 to 10 bar, preferably 1 bar. In any case, the skilled person will be able to set the right values according to the type of instrument he/she uses.

The addition of polyalcohols, particularly mannitol, affords a considerable increase of process yield; indeed, it has been observed that, without polyalcohols, the microparticle yield obtained by *spray-drying* of a mixture of cholesterol, ceramide 3 and C₁₆-C₁₈ fatty acids at a weight ratio of 1:1:1 usually ranges from 14 to 45%, whereas the lipid microparticle yield obtained by *spray-drying* of the same mixture with the addition of 5% to 20% mannitol by weight based on the weight of lipids usually ranges from 45 to 60%. The reason for this is that the polyalcohol can increase the evaporation rate of the solvent, thereby reducing the formation of microparticle aggregates. Furthermore, the particles of the invention were found to have a well-defined mean diameter, usually ranging from 20 to 25 µm, which is particularly suitable for preparing cosmetic and pharmaceutical compositions for topical use.

Therefore, cosmetic or pharmaceutical compositions for topical use which contain the particles of the invention in admixture with suitable excipients or carriers also fall within the scope of the present invention. The microparticles of the invention may be incorporated, for instance, in an amount up to 3% by weight, in emulsions, thereby increasing their occlusive effect without affecting their flowability or structural properties and without leaving any oily feeling.

Particularly, oil-in-water emulsions of the invention may be prepared using the following ingredients.

| **Aqueous phase ingredients** | **Amount (% by weight)** |
|---|---|
| EDTA | 0.1 - 5% |
| Trehalose | 0.1 - 2% |
| Ectoine | 0.1 - 5% |
| Niacinammide | 0.1 - 3% |
| Sodium phytate | 0.1 - 0.5% |
| Chlorophenesin | 0.1 - 0.5 |
| Butylene glycol | 1 - 5% |
| Sodium dehydroacetate | 0.1 - 0.3% |
| Acrylic polymers | 0.1 - 5% |
| Glycerin | 1 - 10% |
| Phenoxyethanol | 0.1- 1% |
| Sodium hyaluronate | 0.1 - 1% |
| Phenyl malonate (HDBM) | 0.01 - 1% |
| Water | Q.s. to 100% |

| **Oily phase ingredients** | |
|---|---|
| Linear esters of fatty acids | 1 - 10% |
| Semisynthetic triglycerides | 1 - 10% |
| Vegetal triglycerides | 1 - 10% |
| Dimethicone | 1 - 10% |
| Phenyl trimethicone | 1 - 10% |
| Medium and long-chain fatty alcohols | 0.1 - 5% |
| Lysophosphatidic acid/lecithin-derived fractions | 0.1 - 5% |
| Polysilicone 11 | 0.1 - 5% |

In any case, cosmetic or pharmaceutical compositions may be prepared using techniques known to the skilled person, such as those described in Remington,

The Science and Practice of Pharmacy, 21st ed., Philadelphia, PA. Lippincott Williams & Wilkins.

The invention and its advantages are described in greater detail in the following experimental section.

### EXPERIMENTAL SECTION

### 1. Materials and Methods

### Preparation and characterization of microparticles

9% w/v lipid solutions were prepared by dissolving cholesterol, ceramide 3 and C₁₆-C₁₈ fatty acids at a weight ratio of 1:1:1 in 100 ml of an 84:16 ethanol/water mixture at a temperature of 80°C.

Mannitol, trehalose and xylitol were added to these solutions, at the following weight ratios to lipids:

| **Polyalcohol** | **lipids/polyalcohol** **weight ratio** |
|---|---|
| Mannitol | 1:1:1:0.6 |
| | 1:1:1:0.45 |
| | 1:1:1:0.3 |
| | 1:1:1:0.15 |
| Threhalose | 1:1:1:0.45 |
| | 1:1:1:0.3 |
| | 1:1:1:0.15 |
| Xylitol | 1:1:1:0.45 |
| | 1:1:1:0.3 |
| | 1:1:1:0.15 |

Then, *spray-drying* was carried out on each solution, using a Mini Spray Dryer B-290 Büchi instrument (Büchi Labortechnick AGF, Switzerland), with the following parameters: inlet temperature: 60°C; outlet temperature: 37°C; gas pressure: 6 bar; aspirator setting: 100% and pump setting: 15%. A 1.5 mm nozzle was used. *Scaling-up* was carried out using a Niro Mobile Minor unit (GEA Inc., Columbia, USA), using the following parameters: inlet temperature (spray nozzle temperature): 80-85°C; outlet temperature: 37-40°C; nitrogen flow rate: 80 kg/h and gas pressure:1 bar. A Co-Fluid nozzle was used.

Particle sizes were measured by laser diffraction particle size analysis (Mastersizer 2000, Malvern, UK) on a water dispersion of microparticles containing 1% polysorbate 60.

### 2. Polyalcohol effect on process yield

The weight-percent yield of the microparticles obtained with the Miny Spray Dryer B-290 Büchi instrument, in the conditions as described under Materials and methods, was measured. The results of a few measurements are set forth in Table 1:

**Table 1**

| **Polyalcohol** | **Lipids/polyalcohol** **weight ratio** | **Weight percent yield** |
|---|---|---|
| **Mannitol** | 1:1:1:0.6 | 53 |
| | 1:1:1:0.45 | 60 |
| | 1:1:1:0.3 | 57 |
| | 1:1:1:0.15 | 45 |
| **Trehalose** | 1:1:1:0.45 | 49 |
| | 1:1:1:0.3 | 40 |
| **Xylitol** | 1:1:1:0.45 | 39 |

Under the same process conditions, the yield of particles prepared from a solution containing cholesterol, ceramide 3 and lipids at a weight ratio of 1:1:1, without polyalcohol, was 45%. The results show that the process of the invention, in which a mixture of cholesterol, ceramide 3, lipids and mannitol, at a weight ratio of 1:1:1:0.45, is used, is particularly advantageous in terms of yield.

### 3. Particle size analysis

Two different batches of microparticles obtained from a solution containing cholesterol, ceramide 3, lipids and mannitol, at a weight ratio of 1:1:1:0.45 were submitted to particle size analysis, at different times, as described under Materials and Methods. The mean diameter of the microparticles was found to range from 20 to 25 µm, with a particle size distribution, expressed in volume distribution units, corresponding to:
d(0,1) = 6.9 µm
d(0,5) = 23.8 µm
d(0,9) = 60 µm

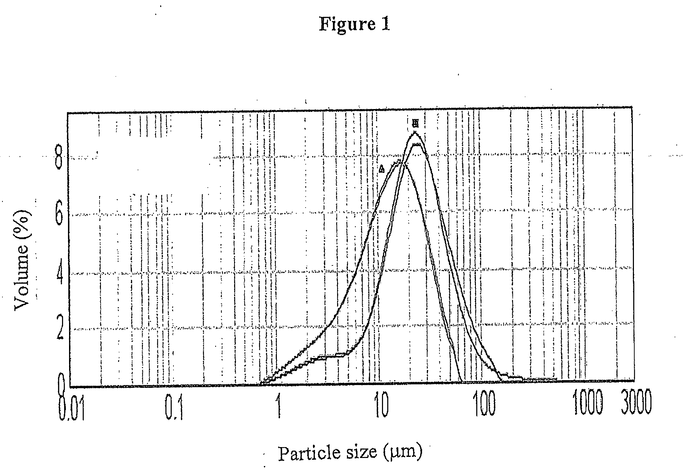

These results are graphically shown in Figure 1, where the two curves designated by ▲ are obtained from measurements made on the first batch at two different times, and the two curves designated by ■ are obtained from measurements made on the second batch at two different times.

### 4. Yield and particle sizes of three different batches obtained using a Niro Mobile Minor unit from a mixture of cholesterol, ceramide 3, lipids and mannitol at a weight ratio of 1:1:1:0.45.

2 kg of a mixture of cholesterol, ceramide 3, lipids and mannitol at a weight ratio of 1:1:1:0.45 were dissolved in 84:12 ethanol:water and submitted to *spray-drying* under temperature flow and nitrogen pressure conditions as set out in Materials and Methods. Inlet and outlet temperatures, and microparticle yields and sizes are set forth in Table 2.

**Table 2**

| **Batch** | **Lipids/mannitol ratio** | **Inlet temperature (°C)** | **Outlet temperature (°C)** | **Process yield** | **Sizes (µm)** |
|---|---|---|---|---|---|
| **1A** | 1:1:1:0.45 | 60 | 37 ÷ 40 | 57% | 1 ÷ 33 |
| **3A** | 1:1:1:0.45 | 70 ÷ 80 | 37 ÷ 40 | 80.6% | 1 ÷ 40 |
| **3A** | 1:1:1:0.45 | 80 ÷ 85 | 37 ÷ 40 | 83.6% | 1 ÷ 45 |

The results show that, on an industrial scale, the yield significantly increases with the increase of the inlet temperature of the mixture.

The particle size analysis of these three batches, that was carried out as described under Materials and Methods, provided the following results:
d(0,1) = 4.5 µm
d(0,5) = 24.4 µm
d(0,9) = 46 µm

### 5. Preparation of emulsions and rheological analyses

An oil-in water emulsion containing, by weight, 2% solid lipid microparticles obtained by *spray-drying* from a solution of cholesterol, ceramide 3, lipids and mannitol at a weight ratio of 1:1:1:0.45, was prepared as disclosed herein, with the microparticles being incorporated in the emulsion at 40°C (sample ELP2). This emulsion was compared with the same emulsion with no lipids (emulsion B1) and with an emulsion in which lipids in non-particle form have been heat-solubilized in the oily phase (sample B2).

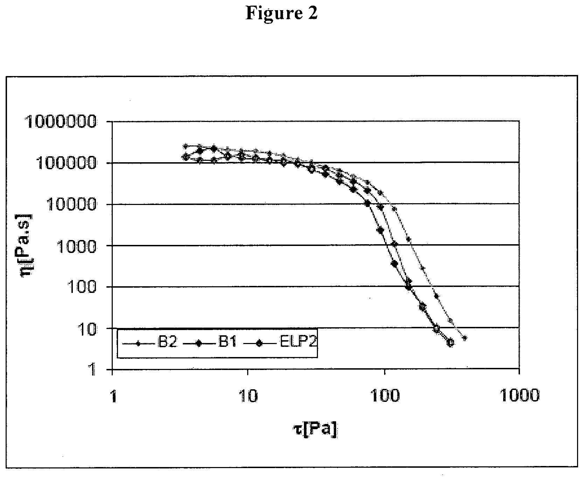

The rheological analyses of the three emulsions have been carried out at 25°C using an Anton Paar Physica MCR-101 rheometer, with a PP50-P2 sensor, and 1 mm gap between the two measuring plates. The chart of Figure 2 shows that all the emulsions have a *pseudoplastic* behavior (with viscosity decreasing with shear), with a Newtonian viscosity *plateau* at the lowest strain values. The presence of the microparticles of the invention increases the critical stress of the emulsion, without affecting the zero-shear viscosity (η₀) as compared with the reference white (sample B1). Conversely, the emulsion containing lipids that have been heat-solubilized before emulsification exhibits higher zero-shear viscosity values (η₀), due to the increase of the internal phase percentage.

### 6. Emulsion effectiveness assessment

Trans Epidermal Water Loss (TEWL) was measured using a Tewameter TM 210® COURAGE & KHAZAKA instrument on 18 healthy volunteers aged from 20 to 45, by applying 2 mg/cm² of each emulsion (ELP2 or B1) on the volar face of the forearm. The TEWL values were recorded before application of the emulsion (T₀) and 30-60-90 minutes after application (T1-T2-T3).

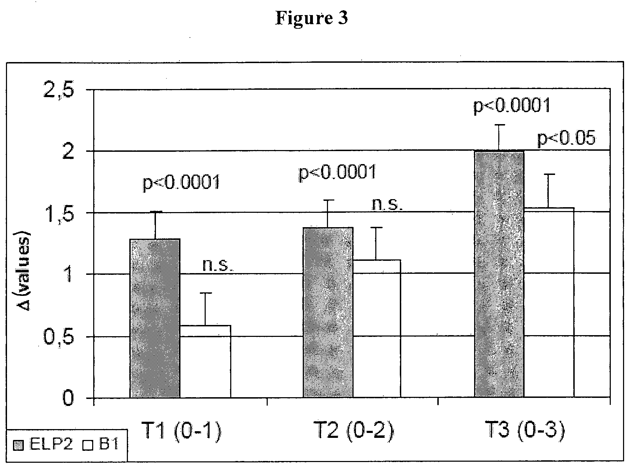

Average reduction of TEWL values (Δ values) after application of the emulsion ELP2 (as determined by Wilcoxon test) was found to be greater and statistically relevant as compared with the corresponding values obtained after application of sample B1 (Figure 3).

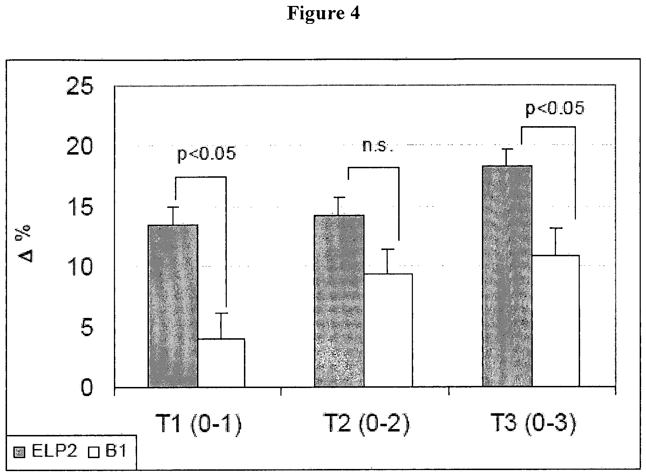

As compared with sample B1, sample ELP2 also showed a fast improvement of the barrier properties of the skin, as confirmed by the determination of TEWL percent reduction (Δ %) at different assessment times (T1-T2-T3) (Mann-Whitney test, U test) (Figure 4).

### 7. Skin roughness analysis

Skin roughness analysis was carried out using an *in-vivo* 3D *scanner* dermaTOP-blue on ten healthy women aged from 40 to 65. The emulsion ELP2 was applied twice a day for one month; skin roughness was assessed at the start of the test (T₀), after 15 days (T₁) and after 30 days (T₂).

Two skin improvement parameters were assessed:
Rz (µm) average maximum profile height difference;
Ra (µm) linear average profile roughness.

As a result of continuous application of the product twice a day, for at least 15 days, an average reduction of the R parameter (roughness) was found. The Rₐ parameter, i.e. an arithmetic mean of the roughness parameters determined on the skin micro-profile, exhibited on average a 18.6% reduction after 15 days and a 20.3% reduction after one-month treatment. The Rz parameter, i.e. the average negative peak value, exhibited on average a 21.7% reduction after 15 days and a 23.0% reduction after one-month treatment.

At both times, the differences between the average values of the 10 volunteers and the T₀ were statistically significant.

## Claims

1. Solid microparticles consisting of epidermal lipids and a polyalcohol selected from mannitol, threhalose and xylitol, wherein the amount of the polyalcohol ranges from 5% to 20% by weight based on the weight of the lipids, and wherein the epidermal lipids are a mixture of cholesterol, one or more ceramides and one or more fatty acids at a weight ratio of 1:1:1.

2. Solid microparticles as claimed in claim 1, wherein the epidermal lipids are a mixture of cholesterol, ceramide 3 and a mixture of C₁₆-C₁₈ fatty acids.

3. Solid microparticles as claimed in claim 1 or 2, wherein the polyalcohol is mannitol.

4. Solid microparticles as claimed in claim 3, wherein mannitol is 15% by weight based on the weight of lipids.

5. A process for preparing solid epidermal lipid microparticles, which includes *spray drying* of an alcoholic solution containing 1 to 20% (w/v) epidermal lipids and 0.1 to 5% (w/v) of mannitol, wherein said epidermal lipids are a mixture of cholesterol, ceramides and one or more fatty acids at a weight ratio of 1:1:1.

6. The process as claimed in claim 5 wherein the alcoholic solution is prepared using an ethanol/water mixture in which the ethanol:water volume ratio ranges from 70:30 to 99:1.

7. The process as claimed in claim 6, wherein the alcoholic solution is a solution that contains 9% (w/v) epidermal lipids and 1.35% (w/v) mannitol in an 84:16 ethanol/water mixture.

8. Use of the microparticles as claimed in any one of claims from 1 to 4, for preparing cosmetic or pharmaceutical compositions.

9. Cosmetic or pharmaceutical compositions containing the microparticles as claimed in any one of claims from 1) to 4), in admixture with suitable excipients and/or carriers.

## Patentansprüche

1. Feste Mikropartikel, bestehend aus epidermalen Lipiden und einem Polyalkohol, ausgewählt aus Mannitol, Trealose und Xylitol, wobei die Menge des Polyalkohols im Bereich von 5 bis 20 Gew.-% liegt, bezogen auf das Gewicht der Lipide, und wobei die epidermalen Lipide eine Mischung aus Cholesterin, einem oder mehreren Ceramiden und einer oder mehreren Fettsäuren in einem Gewichtsverhältnis von 1:1:1 sind.

2. Feste Mikropartikel nach Anspruch 1, wobei die epidermalen Lipide eine Mischung aus Cholesterin, Ceramid 3 und einer Mischung aus C₁₆-C₁₈-Fettsäuren sind.

3. Feste Mikropartikel nach Anspruch 1 oder 2, wobei der Polyalkohol Mannitol ist.

4. Feste Mikropartikel nach Anspruch 3, wobei Mannitol 15 Gew.-%, bezogen auf das Gewicht der Lipide, ist.

5. Verfahren zur Herstellung von festen epidermalen Lipidmikropartikeln, umfassend die Sprühtrocknung einer alkoholischen Lösung, die von 1 bis 20% (Gew./Vol.) epidermale Lipide und von 0,1 bis 5% (Gew./Vol.) Mannitol enthält, wobei die epidermalen Lipide eine Mischung aus Cholesterin, Ceramiden und einer oder mehreren Fettsäuren in einem Gewichtsverhältnis von 1:1:1 sind.

6. Verfahren nach Anspruch 5, wobei die alkoholische Lösung unter Verwendung eines Ethanol/Wasser-Gemisches hergestellt wird, in dem das Ethanol:Wasser-Volumenverhältnis im Bereich von 70:30 bis 99:1 liegt.

7. Verfahren nach Anspruch 6, wobei die alkoholische Lösung eine Lösung ist, die 9% (Gew./Vol.) epidermale Lipide und 1,35% (Gew./Vol.) Mannitol in einem Ethanol/Wasser-Gemisch von 84:16 enthält.

8. Verwendung der Mikropartikel nach einem der Ansprüche 1 bis 4 zur Herstellung von kosmetischen oder pharmazeutischen Zusammensetzungen.

9. Kosmetische oder pharmazeutische Zusammensetzungen, die die Mikropartikel nach einem der Ansprüche 1 bis 4 im Gemisch mit geeigneten Exzipienten und/oder Trägern enthalten.

## Revendications

1. Microparticules solides constituées de lipides épidermiques et d'un polyalcool choisi parmi le mannitol, le tréhalose et le xylitol, la quantité du polyalcool étant comprise entre 5% et 20% en poids par rapport au poids des lipides et les lipides épidermiques étant un mélange de cholestérol, d'un ou de plusieurs céramides et d'un ou de plusieurs acides gras dans un rapport pondéral de 1:1:1.

2. Microparticules solides selon la revendication 1, **caractérisées en ce que** les lipides épidermiques sont un mélange de cholestérol, de céramide 3 et d'un mélange d'acides gras en C₁₆-C₁₈.

3. Microparticules solides selon la revendication 1 ou 2, dans lesquelles le polyalcool est le mannitol.

4. Microparticules solides selon la revendication 3, dans lesquelles le mannitol est 15% en poids par rapport au poids des lipides.

5. Procédé de préparation de microparticules de lipides épidermiques solides, comprenant le séchage par pulvérisation d'une solution alcoolique contenant 1 à 20% (p/v) de lipides épidermiques et 0,1 à 5% (p/v) de mannitol, lesdits lipides épidermiques étant un mélange de cholestérol, de céramides et d'un ou plusieurs acides gras dans un rapport en poids de 1:1:1.

6. Procédé selon la revendication 5, dans lequel la solution alcoolique est préparée en utilisant un mélange éthanol/eau dans lequel le rapport volumique éthanol/eau est compris entre 70:30 et 99:1.

7. Procédé selon la revendication 6, dans lequel la solution alcoolique est une solution contenante 9% (p/v) de lipides épidermiques et 1,35% (p/v) de mannitol dans un mélange éthanol/eau 84:16.

8. Utilisation des microparticules selon l'une quelconque des revendications 1 à 4 pour préparer des compositions cosmétiques ou pharmaceutiques.

9. Compositions cosmétiques ou pharmaceutiques contenantes les microparticules selon l'une quelconque des revendications 1 à 4 en mélange avec des excipients et/ou des véhicules appropriés.
